# EUROPEAN PATENT APPLICATION

(11) **EP 4 667 581 A1**
(43) Date of publication of application: **24.12.2025**
(21) Application number: 24766943.5
(22) Date of filing: 27.02.2024
(51) Int. Cl.: C12Q 1/06, C12M 1/34, G01N 21/27, G01N 21/64, G06T 7/00, G06V 20/69

(54) **CELL IMAGE ANALYSIS METHOD AND PROGRAM**

(30) Priority: 07.03.2023 JP 2023034883
(71) Applicant: Canon Kabushiki Kaisha, Tokyo, 146-8501 (JP)
(72) Inventor: TOBA, Shuhei, Tokyo 146-8501 (JP); HARADA, Ichiro, Tokyo 146-8501 (JP); ICHIMURA, Yoshikatsu, Tokyo 146-8501 (JP)
(74) Representative: WESER & Kollegen Patentanwälte PartmbB
(86) International application number: PCT/JP2024/006992
(87) International publication number: WO 2024/185572

(57) **Abstract**

Provided is a cell image analysis method including: a morphological information image acquisition step of acquiring a morphological information image including morphological information of a cell; an autofluorescence image acquisition step of acquiring an autofluorescence image which is obtained by photographing autofluorescence of the cell and which includes the same field of view as in the morphological information image; a cell region extraction step of extracting one or more cell regions from the morphological information image; and a luminance information extraction step of extracting pieces of luminance information of two or more wavebands different from one another from the autofluorescence image, with respect to the one or more cell regions.

## Description

### [Technical Field]

The present invention relates to a cell image analysis method and a program.

### [Background Art]

In a cell, there are a plurality of substances that emit autofluorescence, and a plurality of coenzymes out of those fluorescent substances are known to indicate information of a redox state and a metabolic state in the cell.

Hasegawa et al. are focusing attention particularly on nicotinamide adenine dinucleotide (NADH) and flavin adenine dinucleotide (FAD) which are coenzymes having a fluorescent brightness that rises and drops depending on the redox state of a living tissue. Hasegawa et al. have developed a system which determines the redox state of a living tissue by calculating a luminance ratio of respective fluorescence wavelengths of NADH and FAD from a captured image of an organ (Non-Patent Literature 1).

With a technology for acquiring autofluorescence information such as the one described in Non-Patent Literature 1, staining-free and non-invasive analysis of a metabolic state and other types of functional information of a cell is expected to become achievable.

Causative substances originating from autofluorescence are coenzymes involved in metabolism and, consequently, by estimating quantities of autofluorescence information of a cell, staining-free estimation of a detailed metabolic state of the cell and a health state of the cell as well as staining-free discrimination of cell species can be achieved.

Fluorescence-activated cell sorting (FACS) is known as a technology for determining quantities of autofluorescence information of a cell. However, FACS is a technology of flow cytometry and, accordingly, is incapable of acquiring information indicating a position and a shape of a cell (hereinafter referred to as "morphological information." FACS is consequently unusable for such uses as analysis and monitoring of autofluorescence information in a cell being cultured.

As a method of non-invasively acquiring morphological information and autofluorescence information of a cell at the same time, an analysis technology using image data is wanted.

In Patent Literature 1, there has been developed a technology for observing morphology and autofluorescence of a cell simultaneously by acquiring a phase difference image and an autofluorescence image, and displaying a composite of the acquired images.

In Patent Literature 2, there has been performed outline extraction that extracts an outline of a cell with use of an image offset from a point of focus (a defocused image).

### [Citation List]

### [Patent Literature]

PTL 1: Japanese Patent Laid-Open No. 2016-161417
PTL 2: Japanese Patent Laid-Open No. 2007-155982

### [Non Patent Literature]

NPL 1: Katsuya Hasegawa, Yasuo Ogasawara, et al., Transactions of the Visualization Society of Japan, 2010, 30, 11, 73-79

### [Summary of Invention]

### [Technical Problem]

With the technology as described in Patent Literature 1, a composite of the phase difference image and the autofluorescence image is displayed, and the morphological information of each cell is not acquired. Accordingly, the technology is incapable of analyzing the autofluorescence information in units of each cell or independent cell clump that is being cultured.

With the technology as described in Patent Literature 2, no autofluorescence image is acquired at the same time as the defocused image and consequently, there is no way of acquiring autofluorescence information of a cell.

The present invention has been made to solve the problems described above. That is, an object of the present invention is to provide a cell image analysis method that enables simultaneous acquisition of morphological information and autofluorescence information of each cell or cell clump in cell culture.

### [Solution to Problem]

According to one aspect of the present invention, there is provided a cell image analysis method characterized by including: a morphological information image acquisition step of acquiring a morphological information image including morphological information of a cell; an autofluorescence image acquisition step of acquiring an autofluorescence image which is obtained by photographing autofluorescence of the cell and which includes the same field of view as in the morphological information image; a cell region extraction step of extracting one or more cell regions from the morphological information image; and a luminance information extraction step of extracting pieces of luminance information of two or more wavebands different from one another from the autofluorescence image, with respect to the one or more cell regions.

Further, according to another aspect of the present invention, there is provided a program for causing a computer to execute the above-mentioned cell image analysis method.

Further, according to still another aspect of the present invention, there is provided a cell image analysis apparatus characterized by including: an image data acquisition unit configured to acquire a morphological information image including morphological information of a cell, and an autofluorescence image which is obtained by photographing autofluorescence of the cell and which includes the same field of view as in the morphological information image; a cell region extraction unit configured to extract one or more cell regions from the morphological information image; and a luminance information extraction unit configured to extract pieces of luminance information of two or more wavebands different from one another from the autofluorescence image, with respect to the one or more cell regions.

### [Advantageous Effects of Invention]

According to the present invention, the cell image analysis method that enables simultaneous acquisition of the morphological information and the autofluorescence information of each cell or cell clump in culture is provided.

### [Brief Description of Drawings]

[Fig. 1]

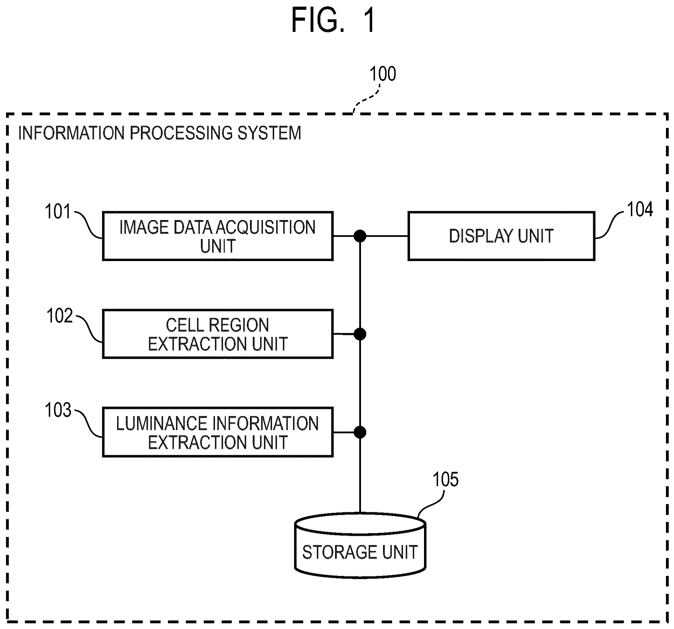

   Fig. 1 is a function block diagram of an information processing system according to a first embodiment.
[Fig. 2]

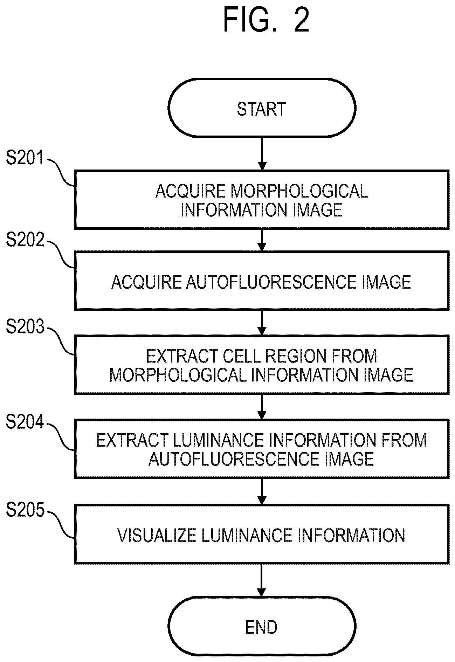

   Fig. 2 is a flow chart of a cell image analysis method according to the first embodiment.
[Fig. 3]

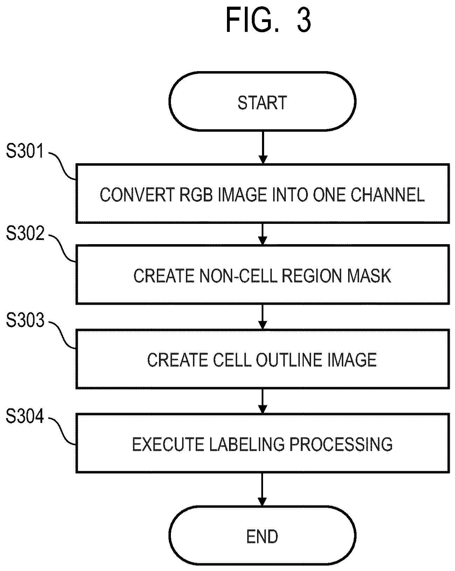

   Fig. 3 is a flow chart for illustrating an example of a method of extracting a cell region in the first embodiment.
[Fig. 4A]

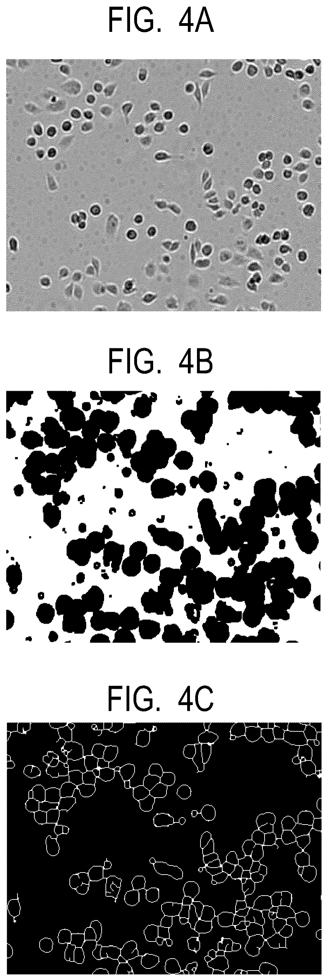

   Fig. 4A is a diagram for showing an example of a grayscale image which is generated by performing grayscaling processing on a defocused image captured with an RGB color camera.
[Fig. 4B]
   Fig. 4B is a diagram for showing an example of a non-cell region mask image which is generated from the grayscale image acquired in Step S301.
[Fig. 4C]
   Fig. 4C is a diagram for showing an example of a cell outline image.
[Fig. 5]

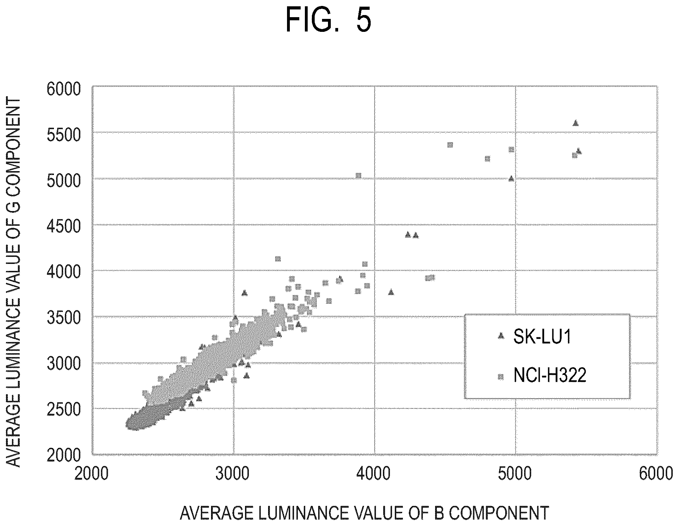

   Fig. 5 is a graph for showing, in the form of a scatter chart, a relationship between a B component and a G component of autofluorescence information of the cell region in Example of the first embodiment.
[Fig. 6]

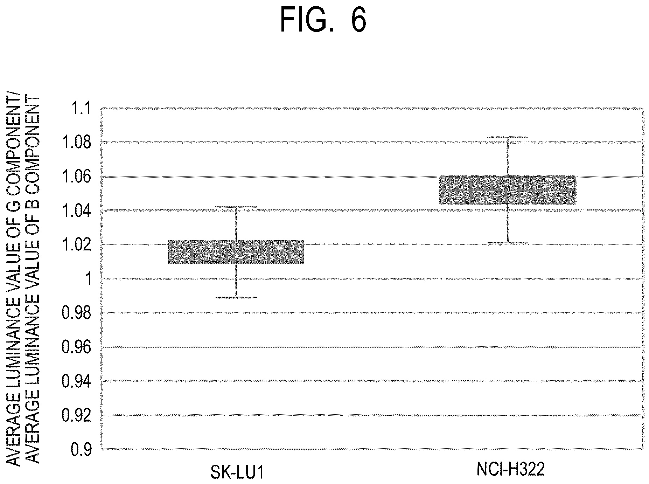

   Fig. 6 is a graph for showing, in the form of a boxplot, a ratio of the B component and the G component of the autofluorescence information of the cell region in Example of the first embodiment.
[Fig. 7]

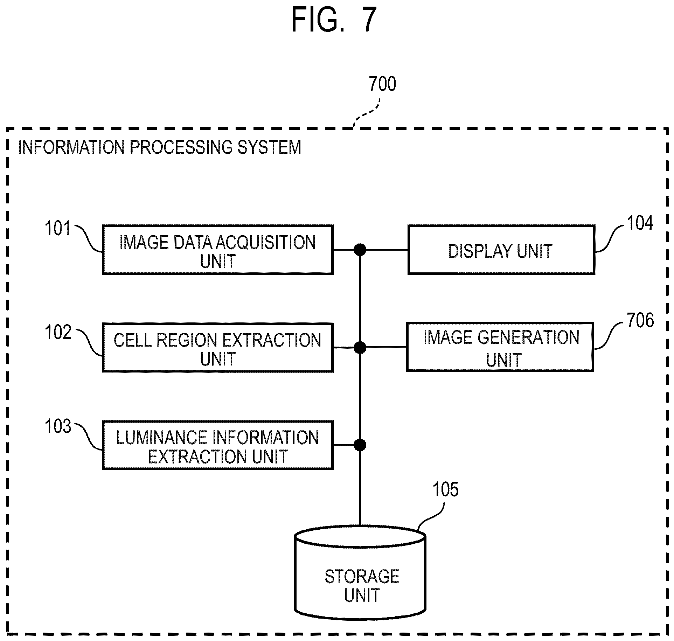

   Fig. 7 is a function block diagram of an information processing system according to a second embodiment.
[Fig. 8]

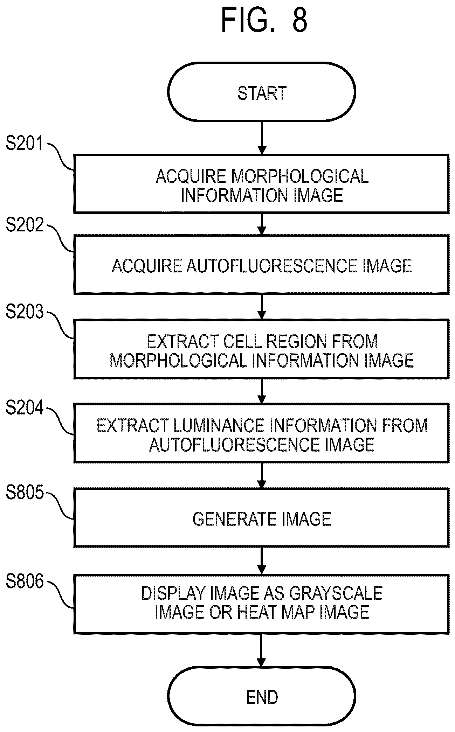

   Fig. 8 is a flow chart of a cell image analysis method according to the second embodiment.
[Fig. 9A]

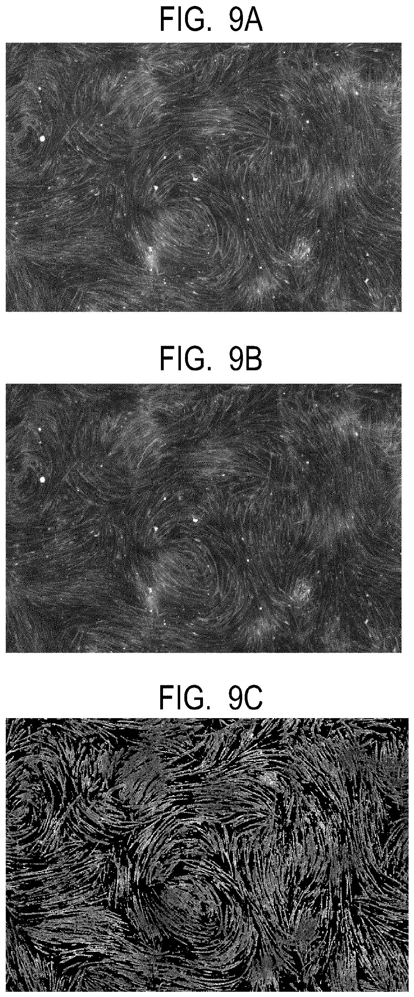

   Fig. 9A is a diagram for showing a result of displaying a G component in grayscale with respect to an image generated based on autofluorescence information in Example of the second embodiment.
[Fig. 9B]
   Fig. 9B is a diagram for showing a result of displaying a B component in grayscale with respect to the image generated based on the autofluorescence information in Example of the second embodiment.
[Fig. 9C]
   Fig. 9C is a diagram for showing a result of displaying, in grayscale, a ratio image generated based on the G component and the B component, with respect to the image generated based on the autofluorescence information in Example of the second embodiment.
[Fig. 10]

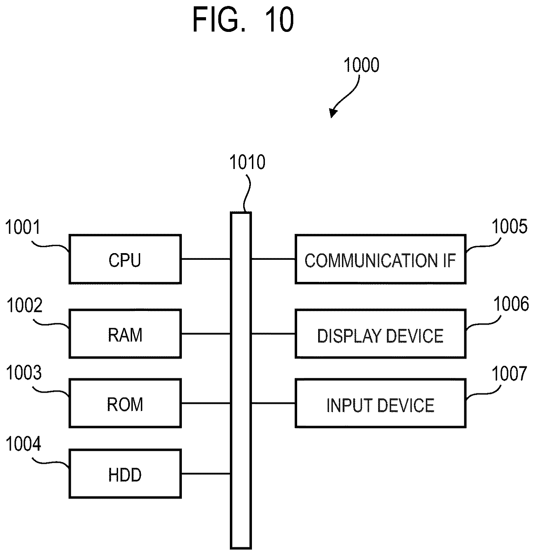

   Fig. 10 is a diagram for illustrating a configuration of an information processing system capable of executing a program according to the present invention.

### [Description of Embodiments]

Now, referring to the accompanying drawings, embodiments of the present invention are described. The present invention is not limited to the following embodiments.

### [First Embodiment]

A cell image analysis method according to a first embodiment is described below.

Fig. 1 is a function block diagram of an information processing system as a cell image analysis apparatus that executes a cell image analysis method and a program according to this embodiment. An information processing system 100 includes an image data acquisition unit 101, a cell region extraction unit 102, a luminance information extraction unit 103, a display unit 104, and a storage unit 105.

### (Image Data Acquisition Unit)

The image data acquisition unit 101 acquires a morphological information image and an autofluorescence image. The morphological information image is an image characterized by having a heightened contrast of an outline portion of a cell to a non-cell region. The autofluorescence image is an image obtained by photographing autofluorescence of a cell that is irradiated with excitation light of a specific wavelength, and is an image containing the same field of view as in the morphological information image described above. Image data acquired by the image data acquisition unit 101 may be image data stored in the storage unit 105 in advance, or may be acquired from an external storage area such as an HDD or cloud storage.

The information processing system 100 of this embodiment may also be connected to a cell image observation apparatus via a communication I/F so as to acquire an image picked up by the cell image observation apparatus.

### (Cell Region Extraction Unit)

The cell region extraction unit 102 extracts one or more cell regions from the morphological information image acquired by the image data acquisition unit 101. To "extract a cell region" means to extract and acquire morphological information of each of cells discretely present in an observation field of view, or to extract and acquire morphological information of each of cells in a cell group that are situated close to one another in a tight mass as well as morphological information of the whole mass.

### (Luminance Information Extraction Unit)

The luminance information extraction unit 103 extracts luminance information as autofluorescence information from the autofluorescence image acquired by the image data acquisition unit 101, with respect to the cell region extracted in the cell region extraction unit 102. The luminance information is information of luminance values corresponding to at least two or more wavebands in a spectrum of autofluorescence that differ from each other.

### (Display Unit)

The display unit 104 displays, on a monitor, the luminance information extracted by the luminance information extraction unit 103, in the form of a histogram, a scatter chart, a boxplot, or the like.

Fig. 2 is a flow chart of the cell image analysis method according to the first embodiment.

A description on details of the respective functions included in the information processing system 100 is given below along with a description on the flow illustrated in Fig. 2.

### (Morphological Information Image Acquisition Step)

In a morphological information image acquisition step of Step S201, the image data acquisition unit 101 acquires the morphological information image containing morphological information of a cell. The morphological information image is, for example, an image of a cell that is obtained at a point of focus, or a bright-field image captured offset from the point of focus by a certain distance along an optical axis direction (hereinafter referred to as "defocused image"). The defocused image is characterized by emphasizing an outline portion of an object such as a cell with a luminance that is high or low compared to a non-cell region. The image to be acquired in this step can be any image characterized by having a heightened contrast of an outline portion of a cell to a non-cell region. To give a specific example, an image picked up with a phase-contrast microscope or an off-axis oblique illumination system, or an image picked up with an optical system between a target object and an image pickup apparatus that is telecentric on the object side and on the image side (a bilateral telecentric optical system) is usable as the morphological information image. It is preferred for the defocused image to be offset from the point of focus to a point at which the contrast of the cell outline portion is most heightened.

### (Autofluorescence Image Acquisition Step)

In an autofluorescence image acquisition step of Step S202, the image data acquisition unit 101 acquires an autofluorescence image of the cell. The autofluorescence image is an image obtained by exciting a cell-intrinsic fluorescent substance via irradiation of the cell with excitation light of a predetermined wavelength, and picking up an image of fluorescence thereof through a filter that cuts light having the wavelength of the excitation light.

The excitation light and the cut filter are preferred to be of a wavelength and a type, respectively, that are suitable for a fluorescent characteristic of an intrinsic component to be observed. For example, in a case in which the intrinsic component is nicotinamide adenine dinucleotide (phosphoric acid) (NAD(P)H), a combination of excitation light of 360 nm and a long-pass filter for 430 nm can be given as an example of the combination of an excitation light wavelength and a cut filter.

In a case in which the intrinsic component is one of flavins (FAD or the like), a combination of excitation light of 450 nm and a long-pass filter for 530 nm can be given as an example.

Other intrinsic components to be observed for autofluorescence include autofluorescent molecules produced within a cell, such as collagen, fibronectin, tryptophan, and folic acid. However, fluorescent intrinsic components other than those may be observed.

The autofluorescence image is an image containing the same field of view as in the morphological information image acquired in Step S201. It is hereinafter assumed that, in this embodiment, the autofluorescence image is captured in the same field of view as that of the morphological information image. The autofluorescence image may be picked up with a camera including a two-dimensional image sensor in which "n" ("n" satisfies n≥2) types of pixels having detection sensitivities to wavebands different from one another are regularly arranged. In this case, luminance information extracted in a luminance information extraction step described later is luminance values recorded by the two-dimensional image sensor.

The two-dimensional image sensor is, for example, a complementary metal-oxide semiconductor (CMOS) sensor as an optical sensor. An example of the camera including the two-dimensional image sensor is an RGB color camera using a CMOS sensor as an RGB color sensor in which three types of pixels are regularly arranged.

However, the optical sensor usable in the present invention is not limited to CMOS sensors and may be, for example, a charge-coupled device (CCD) sensor. In acquisition of the autofluorescence image, a sensor suitable for the wavelength of light emitted by a cell to be observed, or a camera using the suitable sensor, is preferred to be used.

### (Cell Region Extraction Step)

In a cell region extraction step of Step S203, the cell region extraction unit 102 extracts one or more cell regions from the morphological information image acquired in Step S201.

Fig. 3 is a flow chart of cell region extraction in a case in which a defocused image captured with an RGB color camera is acquired as the morphological information image.

### (Cell Region Extraction Processing 1, Conversion to Single Channel)

In Step S301, an RGB image acquired with the RGB color camera is converted into a single channel. For conversion processing, grayscaling processing that is generally used is usable. In a case of an image captured with use of a light source that emits light of a specific waveband, or via a filter that transmits only light of a specific waveband, a channel corresponding to any one component out of R, G, and B may be taken out. Subsequent processing is described on the premise that a grayscale image is generated by grayscaling processing in Step S301.

An example of the grayscale image generated by performing grayscaling processing on the defocused image captured with the RGB color camera is shown in Fig. 4A.

### (Cell Region Extraction Processing 2, Creation of Non-Cell Region Mask Image)

In Step S302, a non-cell region mask image for discriminating a non-cell region in which no cell is present is created. The non-cell region mask image is an image in which pixel values corresponding respectively to the non-cell region and a region other than the non-cell region are expressed by two values different from each other. The two values can be any values: here, the non-cell region is represented by 1 and the other region is represented by 0. The non-cell region mask image is obtained by generating a differential image from the grayscale image acquired in Step S301, and then performing binarization processing.

First, a differential image is generated by applying a differential filter to the grayscale image. The differential image is obtained by calculating, for each pixel, an amount of change in luminance value between the pixel and surrounding pixels, and expressing calculated amounts of change as an image. In a case of a cell image, the differential image is an image that has high luminance values in an outline portion of a cell and interior regions of the cell.

Subsequently, regions having high luminance values in the differential image are extracted by performing binarization processing on the differential image to generate the non-cell region mask image. In the binarization processing, any threshold value is set, and a value of each pixel of the differential image is replaced with 0 when the value is equal to or more than the threshold value, and with 1 when the value is less than the threshold value. How the binarization processing is executed is not limited to the method in which any threshold value is set. For example, a method of automatically determining a threshold value such as Otsu's binarization or binarization by Li's algorithm may be used.

An example of the non-cell region mask image generated from the grayscale image acquired in Step S301 is shown in Fig. 4B.

In the non-cell region mask image, there is a possibility that an undesirable region is interpreted as the cell region or the non-cell region due to an internal structure of the cell, a speck, scarring of a culture vessel, or the like.

In that case, the non-cell region mask image may be modified by morphological processing or hole filling processing.

### (Cell Region Extraction Processing 3, Outline Extraction)

In Step S303, a cell outline image indicating an outline of the cell is generated with use of the grayscale image generated in Step S301.

First, binarization processing is performed on the grayscale image, to thereby generate a mask image for discriminating a region that corresponds to an outline portion of the cell. A threshold value in the binarization processing here may be any value, or may be determined based on a luminance distribution of the grayscale image.

Subsequently, thinning processing is performed on the created mask image. The thinning processing is processing of generating a line image in which a line has a width of one pixel by chipping away a white region of the mask image.

An example of the cell outline image generated from the grayscale image acquired in Step S301 is shown in Fig. 4C.

### (Cell Region Extraction Processing 4, Labeling Processing)

In Step S304, labeling processing of each cell region is executed based on the non-cell region mask image generated in Step S302 and the cell outline image generated in Step S303. In the labeling processing, an image obtained by black-white inversion of the cell outline image is searched for a region to which white pixels are linked, the linked region is labeled as one cell region, and the search and the labeling are repeated.

In labeling, the non-cell region mask image generated in Step S302 is referred to, to thereby exclude the non-cell region from regions to be labeled.

When a boundary between individual cells in the morphological information image is unclear due to the cells being multinuclear cells or the cells forming a cell clump, an independent region formed from a plurality of cells may be labeled as one cell region. In that case, the generation of the cell outline image in Step S303 is omitted, and the labeling processing is executed with the use of the image obtained by inverting the non-cell region mask image. Whether cells are multinuclear cells, or whether cells are forming a cell clump, may be determined by a worker by viewing the morphological information image, or may be determined based on the cell species.

Through the processing steps of from Step S301 to Step S304 described above, each cell region is extracted from the morphological information image. Although the description given above takes, as an example, a flow that uses a defocused image captured with an RGB color camera, the cell region extraction processing is applicable to images in which a contrast of an outline portion of a cell to the non-cell region is heightened. For example, an image acquired with a phase contrast microscope or an image on which image processing is performed so that an outline portion of a cell is emphasized may be used as the image on which the cell region extraction processing is to be executed.

### (Luminance Information Extraction Step)

In the luminance information extraction step of Step S204, the luminance information extraction unit 103 acquires, with regard to the cell region acquired in Step S203, autofluorescence information associated with each cell region from the autofluorescence image acquired in Step S202. Specifically, for each cell region, luminance information of two or more wavebands different from one another is extracted from the autofluorescence image.

As previously mentioned, the autofluorescence image may be picked up with a camera including a two-dimensional image sensor in which "n" ("n" satisfies n≥2) types of pixels having detection sensitivities to wavebands different from one another are regularly arranged. In this case, the luminance information extracted in the luminance information extraction step is luminance values recorded by the two-dimensional image sensor. The luminance information extraction step of Step S204 may include separating data of the autofluorescence image acquired in Step S202 into "n" types of components corresponding to the "n" types of pixels in the two-dimensional image sensor.

The luminance information may also be luminance values recorded by a two-dimensional image sensor that includes a spectral element (for example, a filter, a prism, and a diffraction grating) capable of separating light into light beams of "n" ("n" satisfies n≥2) types of wavebands different from one another, and that includes pixels of one type. In this case, the luminance information extraction step of Step S204 may include separating data of the autofluorescence image acquired in Step S202 into "n" types of components corresponding to the light beams of the "n" types of wavebands.

The luminance information can be extracted by subsequently calculating, for each of the "n" types of components, a luminance statistic which is an average luminance value, a luminance standard deviation, or a luminance median value, in the cell region. The luminance information extraction step may further include calculating a sum, a difference, or a ratio of the luminance statistics of two types of components, out of the luminance statistics of the "n" types of components.

As an example, a case in which an autofluorescence image captured with an RGB color camera is acquired in Step S202 is described.

First, Bayer array data of the autofluorescence image is separated into images having three channels that correspond respectively to an R component, a G component, and a B component. The Bayer array data is two-dimensional array data that is a record of luminance values received by the CMOS color sensor.

The luminance values of the Bayer array data are subsequently separated into components of color filters corresponding to three wavebands, to be separated into the three components which are the R component, the G component, and the B component. Although one pixel has information of the luminance value of one of the R component, the G component, and the B component in the Bayer array data, complementation may be performed so that every pixel has information of the luminance values of the three components which are the R component, the G component, and the B component. Preprocessing such as black level correction processing in which a dark current value is subtracted from each pixel value and filtering processing for noise reduction may be executed.

Subsequently, the luminance information in each cell region acquired in Step S203 is extracted. The luminance information is respective luminance statistics of the R component, the G component, and the B component in each cell region. A luminance statistic is, for example, an average value, a standard deviation, or a median value. The cell image analysis method may be designed so that the luminance statistic is calculated only for a significant component out of the R component, the G component, and the B component, depending on a characteristic of the cut filter that has been used in the capturing of the autofluorescence image.

A sum, a difference, or a ratio of two components out of the three components which are the R component, the G component, and the B component may be calculated to be used as the luminance information of a cell region. For example, a quotient of dividing an average luminance value of the G component by an average luminance value of the B component is calculated.

### (Luminance Information Visualization Step)

In a luminance information visualization step of Step S205, the display unit 104 visualizes the luminance information of each acquired cell region by displaying in the form of a histogram, a boxplot, or the like, or by displaying a relationship between pieces of luminance information of any two components in the form of a scatter chart. The cell analysis method may be designed so as to enable a worker to select any two components, or so as to display every combination of two components.

This concludes the description on a method of simultaneously acquiring morphological information and autofluorescence information of each cell in cell culture and displaying the autofluorescence information of the cell by the steps of from Step S201 to Step S205.

The following is a description about modification examples in the steps of from Step S201 to Step S205 in the first embodiment. Steps to which no mention is made in the respective modification examples are the same as in the first embodiment, and descriptions thereof are accordingly omitted.

### [First Modification Example of First Embodiment]

In Step S202, in a case in which the morphological information image and the autofluorescence image have fields of view different from each other, positioning processing is performed on the morphological information image data and the autofluorescence image data.

The positioning processing is executed by a marking-based method or a method via image processing.

The marking-based method is executed by a worker. In the marking-based method, the worker executes positioning by marking a plurality of points as a position of the same cell in each of the morphological information image and the autofluorescence image, and calculating an affine transformation matrix based on coordinates of the marked points. The number of points to be marked is preferred to be nine or more.

In the case of the method via image processing, positioning is executed with use of, for example, a mask image generated by threshold value processing on the autofluorescence image and the non-cell region mask image generated from the defocused image in Step S302. For positioning of the two mask images, such methods as template matching and a phase-only correlation method are usable.

### [Second Modification Example of First Embodiment]

In Step S202, two or more autofluorescence images different from one another in the combination of the wavelength of the excitation light and a characteristic of the cut filter on the observation side may be acquired. In that case, the luminance information extraction processing in Step S204 is executed for each of the autofluorescence images. This gives each single cell region pieces of luminance information under respective autofluorescence photographing conditions.

### [Third Modification Example of First Embodiment]

The autofluorescence image acquired in Step S202 may be a moving image or a time-lapse image. In that case, the luminance information extraction processing in Step S204 is executed for the autofluorescence image at each photographing time. This gives each single cell region a piece of luminance information for each photographing time.

In a case in which data having the information at the photographing time in this modification example is acquired, a graph for showing changes with time of the respective components and the respective cell regions may additionally be displayed in Step S205.

### [Fourth Modification Example of First Embodiment]

A method of extracting the R component, the G component, and the B component from the Bayer array data in Step S204 is found in the description given above. However, the luminance information may be values obtained by converting the R component, the G component, and the B component into components in a color space or a color specification system different from the RGB color space. Color spaces such as sRGB and Adobe RGB and color specification systems such as Lab, XYZ, and HSV can be given as examples of the color space or the color specification system different from the RGB color space.

### [Fifth Modification Example of First Embodiment]

**In** this embodiment, the luminance information extraction step is described by taking an RGB color camera that uses a CMOS sensor as an example. However, a camera other than an RGB color camera that includes an optical sensor suitable for a wavelength to be observed may be used. That is, an image acquired with the use of, for example, a spectral camera such as a multi-band spectral camera or a hyperspectral camera may be used in the luminance information extraction step. Examples of the usable optical sensor include a sensor that uses Si, Ge, and InGaAs as materials forming the sensor, and a sensor having such a sensor structure as a CMOS-type structure, a CCD-type structure, an avalanche photodiode array (APD)-type structure, or a bolometer-type structure.

In that case, in Step S204, the acquired image is separated into images of a plurality of components corresponding to a plurality of wavelength filters included in the camera.

### [Example of First Embodiment]

The cell image analysis method according to the first embodiment is usable in cell culture in which a clear boundary is depicted between cells in a morphological information image, such as culturing of cancer cells. An example in which a human lung cancer cell SK-LU1 and a human lung cancer cell NCI-H322 are targets is described below.

### (Target Cells and Culture)

The following two samples were prepared.
· A sample obtained by culturing a human lung cancer cell SK-LU1 in Cell Culture Medium No. 104 (a product of KAC Co., Ltd.)
· A sample obtained by culturing a human lung cancer cell NCI-H322 in Cell Culture Medium No. 103 (a product of KAC Co., Ltd.)

A 35-mm glass bottom dish was used as a cell culture vessel.

### (Morphological Information Image)

A defocused image offset from the point of focus by -100 µm was acquired with an RGB color camera including, among others, a CMOS sensor. The image was picked up via a bilateral telecentric lens.

### (Autofluorescence Image)

An image was acquired by irradiating each sample with excitation light from a 365-nm LED light source as epi-illumination from the image pickup side, and photographing resultant fluorescence through a long-pass filter for 430 nm, with the same RGB color camera that was used for the morphological information image, in the same field of view as that of the morphological information image.

### (Luminance Information Extraction Processing)

The R component, the G component, and the B component of the Bayer array data were acquired. In each cell region, three types of luminance information which were an average luminance value of the G component, an average luminance value of the B component, and a quotient of dividing the average luminance value of the G component by the average luminance value of the B component were calculated.

### (Display of Autofluorescence Information)

A graph in which a relationship between the average luminance value of the B component and the average luminance value of the G component in each cell region is plotted in the form of a scatter chart is shown in Fig. 5. The axis of ordinate indicates the average luminance value of the G component, and the axis of abscissa indicates the average luminance value of the B component. Dark gray marks represent the NCI-H322 cell sample, and light gray marks represent the SK-LU1 cell sample. Distributions are slanted differently from each other, which suggests that the difference in cell species manifests as a difference in luminance between wavelength components of an autofluorescence spectrum.

When it is indicated that a difference in state of cells between experiment samples appears on the luminance relationship between specific components of the autofluorescence spectrum in this manner, it is effective to calculate a ratio of the two components of each cell and display the ratio in the form of a boxplot or in a similar format. A result of creating a boxplot with respect to the luminance information that is the quotient of dividing the average luminance value of the G component by the average luminance value of the B component is shown in Fig. 6.

This concludes the example in a case of two types of lung cancer cells as a preferred Example of the cell image analysis method according to the first embodiment. The first embodiment is not limited to cancer cells, and is usable for culturing of a normal cell, mixed culturing of a cancer cell and a normal cell, mixed culturing of cancer cells, mixed culturing of normal cells, and a piece of tissue in which those are present in a mixed manner.

### [Second Embodiment]

In the first embodiment, a method in which autofluorescence information associated with each cell region is extracted and the autofluorescence information of each cell is displayed in the form of a histogram, a boxplot, a scatter chart, or the like has been described.

In this embodiment, a method in which a ratio image is created based on luminance information of autofluorescence information, and a grayscale image or a heat map image that visualizes a local change in relationship between components of the autofluorescence information within an image pickup field is displayed is described.

Fig. 7 is a function block diagram of an information processing system that executes a cell image analysis method and a program according to the second embodiment. An information processing system 700 includes the image data acquisition unit 101, the cell region extraction unit 102, the luminance information extraction unit 103, the display unit 104, the storage unit 105, and a ratio image generation unit 706.

The image data acquisition unit 101, the cell region extraction unit 102, the luminance information extraction unit 103, and the storage unit 105 are the same as in the first embodiment, and descriptions thereof are accordingly omitted here.

### (Image Generation Unit)

The image generation unit 706 generates, based on the cell regions extracted in the cell region extraction unit 102 and the luminance information extracted in the luminance information extraction unit 103, an image indicating a change within an image pickup field with respect to a relationship between pieces of luminance information of two or more wavebands different from one another. For example, a ratio image obtained by calculating a ratio of pieces of luminance information in two wavebands different from each other is generated.

### (Display Unit)

In this embodiment, the display unit 104 displays the image generated by the image generation unit 706 as a grayscale image or a heat map image, on a monitor or the like.

Fig. 8 is a flow chart of a cell image analysis method according to a second embodiment.

A description on details of the respective functions included in the information processing system 700 is given below along with a description on the flow of Fig. 8.

Step S201, Step S202, Step S203, and Step S204 are the same as Step S201 to Step S204 in the first embodiment, respectively, and descriptions thereof are accordingly omitted.

### (Image Generation Step)

In an image generation step of Step S805, the image generation unit 706 generates, based on the luminance information extracted in Step S204, an image indicating a change within the image pickup field with respect to a relationship between pieces of luminance information of two or more wavebands different from one another. Here, a case of generating a ratio image by calculating a ratio of pieces of luminance information in two wavebands different from each other is described.

As an example, a case in which an autofluorescence image captured with an RGB color camera is acquired in Step S202 as in the first embodiment is described.

First, a ratio image is created by calculating a ratio of images of any two channels out of the images of three channels obtained in Step S204 by separation into the R component, the G component, and the B component. The selection of any two channels can be based on a characteristic of a cut filter used in capturing of the autofluorescence image. For example, in a case of an autofluorescence image obtained by photographing, through a long-pass filter for 430 nm, a sample irradiated with excitation light of 360 nm, the B component and the G component are selected. In that case, an image obtained by calculating, for each pixel, a quotient of dividing a luminance value of the G component by a luminance value of the B component is the ratio image.

The creation of the ratio image may be executed after noise reduction processing such as average value filtering or median value filtering is executed for each component.

### (Ratio Image Display Step)

In an image display step of Step S806, the display unit 104 displays, in the form of a grayscale image or a heat map image, the ratio image generated in Step S805, on a monitor or the like. The grayscale image is an image in which a magnitude of a pixel value of the ratio image is expressed in shades between black and white. The heat map image is an image in which a specific color is assigned to a specific pixel value of the ratio image based on the magnitude of the pixel value.

Step S806 may be designed so that only cell regions are displayed based on the cell regions extracted in Step S203. In that case, the non-cell region is displayed by, for example, black pixels.

A dynamic range in which the ratio image is displayed may be designed so as to be adjustable by a worker by concurrently displaying a distribution of pixel values of the ratio image as a histogram, or may be determined automatically based on the distribution of the pixel values. In the case in which the dynamic range is automatically determined, a minimum value and a maximum value are determined by, for example, using a mode value as a reference and ensuring that the region takes up 80% of the number of pixels of the cell region.

The following is a description about modification examples of the second embodiment. Steps to which no mention is made in the respective modification examples are the same as in the second embodiment, and descriptions thereof are accordingly omitted.

### [First Modification Example of Second Embodiment]

In Step S805, the ratio image is created by calculating the ratio of pixel values of any two channels. However, the ratio image may be created by calculating, for each cell region, a ratio of a luminance statistic of the cell region and mapping the calculated ratio to pixels that correspond to the cell region. For example, a value that is a quotient of dividing an average luminance value of the G component by an average luminance value of the B component is calculated in a cell region, the calculated value is assigned to every pixel value associated with the cell region, and the calculation and the assigning are executed for every cell region.

### [Second Modification Example of Second Embodiment]

In Step S805, the ratio image is created by calculating the ratio of pixel values of any two channels. However, how a local change in relationship between components of autofluorescence information is visualized is not limited to the method in which the ratio is calculated. For example, the image indicating a change within the image pickup field with respect to a relationship between pieces of luminance information of two or more wavebands different from one another may be an image in which a sum or a difference calculated for any two channels is assigned as a pixel value.

### [Example of Second Embodiment]

A result of using a skeletal muscle cell as a target is described below as a preferred Example of the cell image analysis method according to the second embodiment.

### (Target Cell and Culture)

Growth culture of a C2C12 cell which is a mouse myoblast cell was performed in a DMEM containing 10% fetal bovine serum until a confluence of 90%. The DMEM was subsequently replaced with a DMEM containing 2% equine serum and, after five days of culture with a culture medium replaced with the same type of culture medium every second day, myotube formation progressed to yield differentiation-induced skeletal muscle cells. This differentiation induction induces a state in which a slow-muscle-like skeletal muscle cell which expresses myosin unique to slow muscle and a fast-muscle-like skeletal muscle cell which expresses myosin unique to fast muscle are mixed. It has been known that, because slow muscle expresses mitochondria in a larger amount compared to fast muscle, which metabolic pathway is dominant varies between glycolysis and respiration. Accordingly, a difference in luminance information is expected to be non-uniform among the differentiated skeletal muscle cells.

### (Morphological Information Image)

A defocused image offset from the point of focus by -100 µm was acquired with an RGB color camera including, among others, a CMOS sensor. The image was picked up via a bilateral telecentric lens.

### (Autofluorescence Image)

An image was acquired by irradiating the sample with excitation light from a 365-nm LED light source as epi-illumination from the image pickup side, and photographing resultant fluorescence through a long-pass filter for 430 nm, with the same RGB color camera that was used for the morphological information image, in the same field of view as that of the morphological information image.

### (Generation and Display of Ratio Image)

A ratio image was generated by calculating a quotient of dividing the luminance value of the G component by the luminance value of the B component in the autofluorescence image. Fig. 9A is a result of displaying the G component in grayscale, and Fig. 9B is a result of displaying the B component in grayscale. Fig. 9C is a result of displaying, in grayscale, the ratio image generated based on the G component and the B component. In the ratio image, the non-cell region is displayed as black pixels.

As shown in Fig. 9A to Fig. 9C, components of an autofluorescence spectrum of the differentiated skeletal muscle cells are non-uniform in the overall culture system, and a domain different in metabolic state after the differentiation is discriminable. In addition, a difference in metabolic state reflects a difference of skeletal muscle species after the differentiation, and cell species can accordingly be discerned from one another by using the cell image analysis method according to the second embodiment.

This concludes the description on the result of application to a skeletal muscle cell as a preferred Example of the cell image analysis method according to the second embodiment. This embodiment is not limited to skeletal muscle cells, and is usable for multinuclear cells such as skeletal muscle and liver cells, or cells forming a cell clump.

Any of the embodiments described above is merely an example of how the present invention is carried out in a practical manner, and is not to be a basis for limited interpretations of a technical scope of the present invention. That is, the present invention can be carried out in various forms without departing from a technical concept or main features of the present invention.

### <Program>

A program according to the present invention is a program for causing a computer to execute the cell image analysis method according to the present invention described above.

Fig. 10 is a block diagram for illustrating a hardware configuration example of an information processing system serving as the cell image analysis apparatus in which the program according to the present invention is executable.

An information processing system 1000 has functions of a computer. For example, the information processing system 1000 may be configured unitarily with a desktop personal computer (PC), a laptop PC, a tablet PC, or a smartphone, for example.

The information processing system 1000 includes, in order to implement functions as a computer which performs arithmetic operation and storage, a central processing unit (CPU) 1001, a random-access memory (RAM) 1002, a read-only memory (ROM) 1003, and a hard disk drive (HDD) 1004. The information processing system 1000 also includes a communication interface (I/F) 1005, a display device 1006, and an input device 1007. The CPU 1001, the RAM 1002, the ROM 1003, the HDD 1004, the communication I/F 1005, the display device 1006, and the input device 1007 are connected to each other via a bus 1010. The display device 1006 and the input device 1007 may be connected to the bus 1010 via a drive device (not shown) for driving those devices.

In Fig. 10, the various components forming the information processing system 1000 are illustrated as an integrated device, but a part of the functions of those components may be implemented by an external device. For example, the display device 1006 and the input device 1007 may be external devices different from the components implementing the functions of the computer including the CPU 1001, for example.

The CPU 1001 performs predetermined operations in accordance with programs stored in, for example, the RAM 1002 and the HDD 1004, and also has a function of controlling each component of the information processing system 1000. Processing executed by the CPU may include acquisition of the morphological information image and the autofluorescence image, extraction of cell regions, extraction of luminance information, generation of the ratio image, and the like.

The RAM 1002 is built from a volatile storage medium, and provides a temporary memory area required for the operations of the CPU 1001. The ROM 1003 is built from a non-volatile storage medium, and stores required information such as programs to be used for the operations of the information processing system 1000.

The HDD 1004 is a storage device which is built from a non-volatile storage medium, and which stores the morphological information image, the autofluorescence image, the luminance information, and the like.

The communication I/F 1005 is a communication interface based on a standard such as Wi-Fi (trademark) or 4G, and is a module for communicating to and from another device. The display device 1006 is, for example, a liquid crystal display or an organic light emitting diode (OLED) display, and is used for displaying moving images, still images, and characters, for example. The input device 1007 is, for example, a button, a touch panel, a keyboard, or a pointing device, and is used by a user to operate the information processing system 1000. The display device 1006 and the input device 1007 may be integrally formed as a touch panel.

The hardware configuration illustrated in Fig. 10 is an example, and devices other than the illustrated devices may be added, or a part of the illustrated devices may be omitted. Further, a part of the devices may be substituted with another device having the same function. Moreover, a part of the functions may be provided by another device via a network, and the functions for implementing the embodiments may be shared and implemented by a plurality of devices. For example, the HDD 1004 may be substituted with a solid state drive (SSD) which uses a semiconductor element, such as a flash memory, or may be substituted with cloud storage.

The present invention is not limited to the embodiments described above, and various changes and modifications can be made thereto without departing from the spirit and scope of the present invention. The following claims are appended hereto in order to make the scope of the present invention public.

The present application claims priority based on Japanese Patent Application No. 2023-034883 filed on March 7, 2023, and the entire contents thereof are incorporated herein by reference.

### [Reference Signs List]

- 100, 700, 1000: information processing system (cell image analysis apparatus)
- 101: image data acquisition unit
- 102: cell region extraction unit
- 103: luminance information extraction unit
- 104: display unit
- 105: storage unit
- 706: image generation unit

## Claims

1. A cell image analysis method comprising:
a morphological information image acquisition step of acquiring a morphological information image including morphological information of a cell;
an autofluorescence image acquisition step of acquiring an autofluorescence image which is obtained by photographing autofluorescence of the cell and which includes the same field of view as in the morphological information image;
a cell region extraction step of extracting one or more cell regions from the morphological information image; and
a luminance information extraction step of extracting pieces of luminance information of two or more wavebands different from one another from the autofluorescence image, with respect to the one or more cell regions.

2. The cell image analysis method according to claim 1, wherein the pieces of luminance information are luminance values recorded by a two-dimensional image sensor in which "n" types of pixels having detection sensitivities to wavebands different from one another are regularly arranged, where "n" satisfies n≥2.

3. The cell image analysis method according to claim 2, wherein the luminance information extraction step includes separating data of the autofluorescence image into "n" types of components corresponding to the "n" types of pixels in the two-dimensional image sensor.

4. The cell image analysis method according to claim 3, wherein the luminance information extraction step includes, when the two-dimensional image sensor is an RGB color sensor in which three types of pixels are regularly arranged, separating the data of the autofluorescence image into three components which are an R component, a G component, and a B component.

5. The cell image analysis method according to claim 4, wherein the pieces of luminance information are values obtained by converting the three components which are the R component, the G component, and the B component into components of a color space or a color specification system that is different from an RGB color space.

6. The cell image analysis method according to claim 1, wherein the pieces of luminance information are luminance values recorded by a two-dimensional image sensor which includes a spectral element that separates light into light beams of "n" types of wavebands different from one another, where "n" satisfies n≥2, and which includes pixels of one type.

7. The cell image analysis method according to claim 6, wherein the luminance information extraction step includes separating data of the autofluorescence image into "n" types of components corresponding to the light beams of the "n" types of wavebands.

8. The cell image analysis method according to any one of claims 3 to 5 and 7, wherein the luminance information extraction step includes calculating, for each of the "n" types of components, an average luminance value, a luminance standard variation, or a luminance median value which is a luminance statistic, in each of the one or more cell regions.

9. The cell image analysis method according to claim 8, wherein the luminance information extraction step includes calculating a sum, a difference, or a ratio of the luminance statistics of two types of components out of luminance statistics of the "n" types of components.

10. The cell image analysis method according to any one of claims 2 to 9, wherein the two-dimensional image sensor is a CMOS sensor.

11. The cell image analysis method according to any one of claims 1 to 10, further comprising:
an image generation step of generating, based on the pieces of luminance information, an image indicating a change within an image pickup field with respect to a relationship between the pieces of luminance information of the two or more wavebands different from one another; and
an image display step of displaying the image generated in the image generation step as a grayscale image or a heat map image.

12. The cell image analysis method according to any one of claims 2 to 10, further comprising:
an image generation step of generating, based on the pieces of luminance information, an image indicating a change within an image pickup field with respect to a relationship between the pieces of luminance information of the two or more wavelengths different from one another; and
an image display step of displaying the image generated in the image generation step as a grayscale image or a heat map image,
wherein the image generation step includes generating an image that has, as a pixel value, a value obtained by calculating a sum, a difference, or a ratio of luminance values of two components out of the "n" types of components corresponding to respective pixels.

13. A program for causing a computer to execute the cell image analysis method of any one of claims 1 to 12.

14. A cell image analysis apparatus comprising:
an image data acquisition unit configured to acquire a morphological information image including morphological information of a cell, and an autofluorescence image which is obtained by photographing autofluorescence of the cell and which includes the same field of view as in the morphological information image;
a cell region extraction unit configured to extract one or more cell regions from the morphological information image; and
a luminance information extraction unit configured to extract pieces of luminance information of two or more wavebands different from one another from the autofluorescence image, with respect to the one or more cell regions.

15. The cell image analysis apparatus according to claim 14, further comprising an image generation unit configured to generate an image indicating a change within an image pickup field with respect to a relationship between the pieces of luminance information of the two or more wavebands different from one another based on the pieces of luminance information.
